# EUROPEAN PATENT SPECIFICATION

(11) **EP 0 506 619 B1**
(45) Date of publication and mention of the grant of the patent: **19.07.1995**
(21) Application number: 92830068.0
(22) Date of filing: 18.02.1992
(51) Int. Cl.: A22B 5/00, A61B 5/05

(54) **A device for identifying and evidencing distinct anatomical regions in animals, in particular specific points in the limb joints**
Vorrichtung zur Erkennung und Markierung von bestimmten an anatomischen Gegenden bei Tieren, insbesondere spezifischen Stellen in Gliedgelenken
Appareil pour identifier et marquer des zones anatomiques distinctes d'animaux, en particulier des points spécifiques dans les joints des membres

(30) Priority: 29.03.1991 IT BO910103
(43) Date of publication of application: 30.09.1992
(73) Proprietor: EUROPORK S.P.A., I-41057 Spilamberto (Modena) (IT)
(72) Inventor: Cavilli, Gianfranco, I-41057 S. Vito di Spilamberto, (Modena) (IT)
(74) Representative: Lanzoni, Luciano

(56) References cited:
- EP-A- 0 212 580
- EP-A- 0 273 371
- GB-A- 2 019 579
- GB-A- 2 117 224
- US-A- 3 882 570
- US-A- 3 940 998
- US-A- 4 486 835
- US-A- 4 920 490

## Description

The invention relates to a device capable of identifying and evidencing distinct anatomical regions in animals, and in particular, as we will show in the following text, specific points in limb joints.

At present the identifying of both distinct anatomical regions and specific points in the limb joints of animals, for example during butchering and successive working of the meats destined for consumption, is done empirically and entirely manually, for example by compressing the region where the joint is thought to be and performing a series of manipulations dictated entirely by the experience of the operator, which manipulations allow the said operator to understand where the specific points are.

The identification of the specific points of the joints is important especially in the working of pigmeats where, for example, it is necessary to separate the terminal part of the posterior limb or thigh in order to define the ham as the region between the cuboid and the metatarsus. Such a separation, in order to be considered properly performed, must be realised by means of a single cut, a cut which can be made only at the point of the joint where there are no hard parts such as bones but only soft parts, that is, the cartilage which separates the bones. Thus it is evident how important this cutting operation is if the ham is to be considered properly worked, and even an expert can take a considerable amount of time to perform it satisfactorily. It is also evident that an accurate cutting operation also eliminates problems constituted by possible bone splinters which, by getting into the meat itself, could ruin it.

Another important point is that the time needed to perform the delicate cutting operation is not compatible with the needs of modern industrial practice, nor with the speeds possible with modern machinery already available at other points in the working of the hams, for example the rapid boning machines already in use.

A method and an apparatus for severing a portion of an animal leg is disclosed in the American Patent US-A-3 882 570 (Zwiep). The apparatus includes a pairs of rollers mounted in a pivoting frame. The roller moving along the leg of the animal, for example in the area of the heel, can sense the position for the cut, making to swing the pivoting frame. A limit switch actioned by the pivoting frame sends a signal to a cutter in order to sever the portion of animal leg in the requested position. This mechanical sensor formed by these rollers does not permit the necessary flexibility in order to perform a cut in a different joint of the animal, but requires always a particular form of the animal leg, such as in the heel area, to make to swing the pivoting frame. It is therefore necessary to substitute this mechanical system with a different operated system able to detect the joint.

Another document, the British Patent GB-A-2 019 579 (Philips), discloses a method and a device for determining the internal structure of a body.
An electric field is applied to different sectional plane of the body, this cart be obtained either by a physical rotation of the electrode array or by an electrical connection of one of the electrode arrays that fully encircled the body part. The measurement of the path resistance values then occuring, permits, by means of matematical calculation, to build the internal structure of the body.

Departing from these two documents, the aim of the present invention is therefore to provide a device to identify and evidence the limb joints of animal parts in a simple and sure way, to permit the automisation of this operation, and eliminate the above-described drawbacks.

The invention, as described in the claim which follow, resolves the problem with a device for the identifying and evidencing of anatomical regions of animals, in particular specific points in the limb joints, characterised by the fact that it essentially comprises:
- support means for at least the part of the animal of which the distinct anatomical region or the specific limb joint has to be identified;
- at least two sensors arranged on opposite sides of the said animal part able to identify at least one characteristic or the physical size of the said anatomical region of the animal part to be identified;
- means to evidence the said identified anatomical region;
- a processing unit placed in communication with the said sensors and said evidencing means which unit is able to process the data received from the said sensors and to command the activation of the said evidencing means according to the data received from the said sensors.

The characteristics and advantages of the present invention will better emerge from the detailed description which follows, made with reference to the accompanying drawings which are included in the form of a non-limiting example, and in which:
- Figure 1 shows the device of the present invention, schematically and in plan view, with several parts removed better to illustrate others;
- Figure 2 shows, schematically in front view, and with some parts removed better to evidence others, the device of figure 1;
- Figure 3 shows the transversal section of a cursor of the device of the previous illustrations;
- Figure 4 shows the block diagram of the device of figures 1 and 2.

The device to identify and evidence anatomical regions in animal parts, in particular specific points in the limb joints of the said animals, is denoted generically by 1 in the figures: it comprises essentially support means 3 for the animal part 2 to be examined, which hereinafter will be referred to simply as the limb, at least two sensors 4 and 5, able to evidence the characteristics or the physical size of the joint limb 2, of means 6 to evidence a region of the limb 2 and a processing unit 7 able to manage both the sensors 4 and 5 and the signals transmitted by them and the evidencing means 6 and the support means 3. The support means 3 consist of a plane 26 on which the limb 2 is laid and of a pair of parallel slide bars 27 supporting the said plane 26. Movement means 28, commanded by the processing unit 7, act on the said plane 26, moving it along the said slide bars 27. The said movement means 28 can bring the said support plane 26 into a plurality of positions along the said slide bars 27 and can be constituted by for example, an electrically-powered linear actuator. At one end of the said slide bars 27 sensors 4 and 5 are envisaged, positioned on opposite sides of the vertical plane parallel to the slide bars 27 themselves and in all intermediate position with regard to them.

In the illustrated embodiment, which is in particular specifically predisposed to identify the specific points of the pig foot-leg joint, in order successively to perform the cutting operation and thus define the ham, the sensors 4 and 5 are able to calculate the transversal electrical resistance offered by the limb 2. It is known that in the joints regions a relatively high quantity of mineral salts is present, dissolved in the synovial fluid which lubricate and protect the bone parts subject to friction. This liquid constitutes thus a preferential passageway for the electric current induced by measured impulses and determines a low resistance reading.

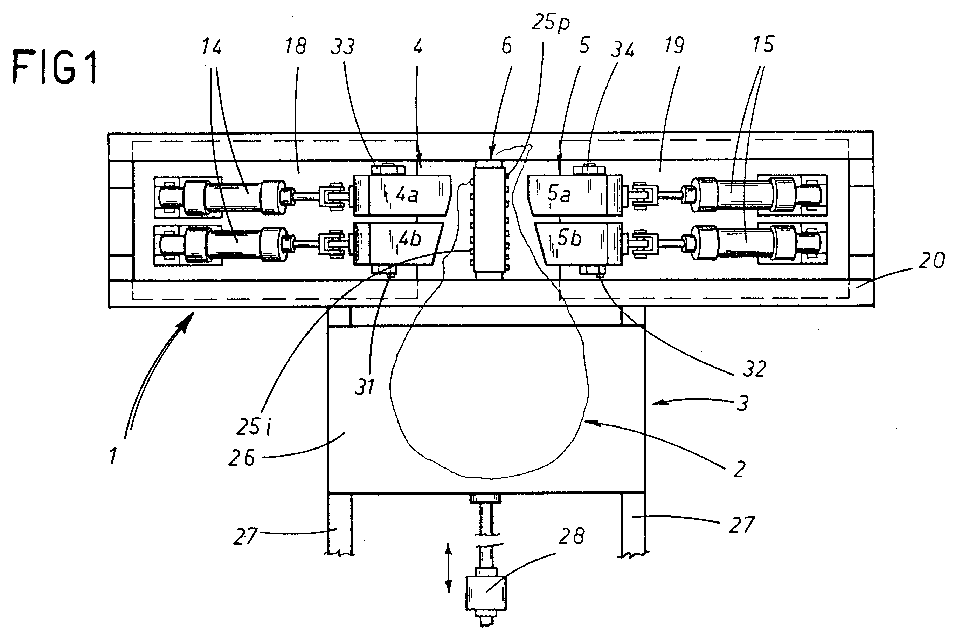

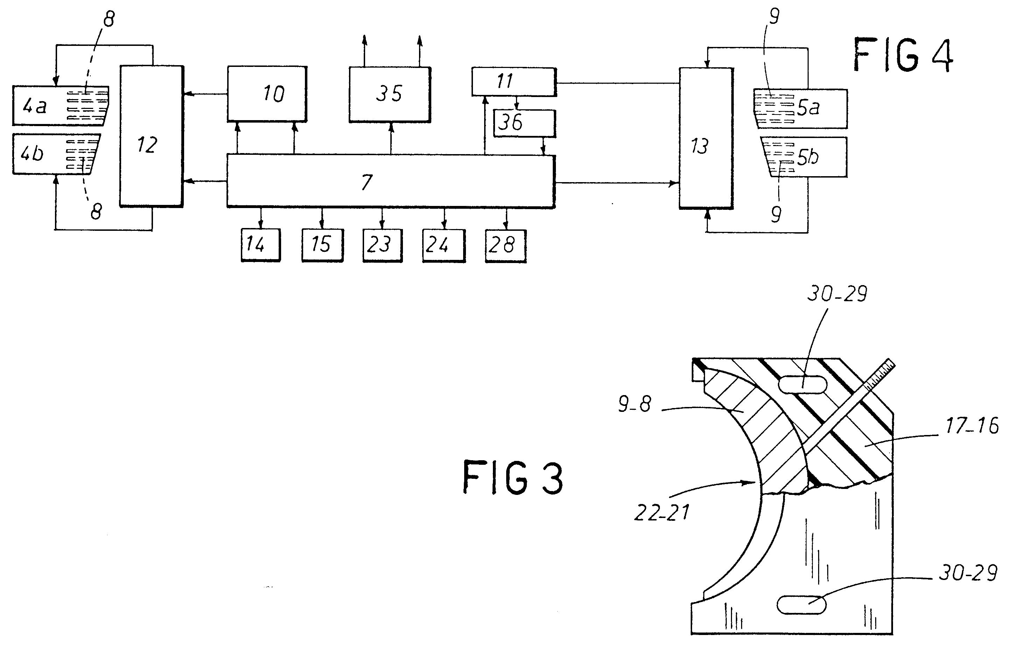

Looking at the figures, the sensors 4 and 5 are constituted by at least two groups 4 and 5 of electrodes 8 and 9, side-by-side and destined to come into opposite contact with the limb 2. In figure 4 eight electrodes 8 are schematically represented, as well as eight more electrodes 9, although the number of the said electrodes can vary according to the working field of the device 1 object of this invention. Groups 4 and 5 are provided in two couples in figure 1, where they are denoted by 4a, 4b, 5a and 5b. The electrodes 8 of groups 4a and 4b are connected to an impulse generator 10 through a respective selector 12, while the electrodes 9 of the groups 5a and 5b are connected to an impulse receiver 11 through a respective selector 13 (see in particular figure 4).

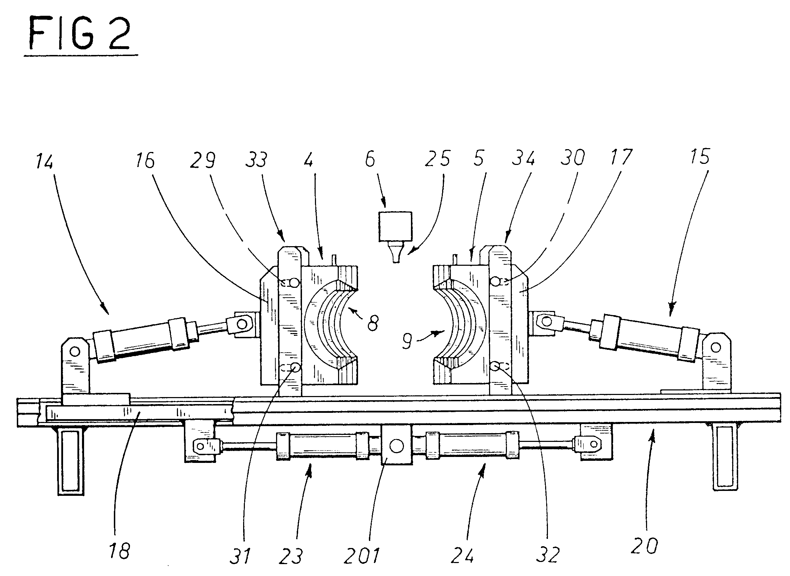

Each electrode 8 or 9 has a substantially half-moon form (see figure 3) and has its concave tract, indicated by 21 and respectively by 22, facing the concave tract 22 or 21 of the electrode 9 or 8 which faces it in the absence of the limb 2. In figure 2 it can be seen that the angle of curvature of the electrodes 8 and 9 decreases as it gets further away from the plane 26, substantially with respect to the shape of the limb 2. The number of electrodes 8 is preferably equal to the number of the electrodes 9 and each electrode 8 is coplanar to a corresponding electrode 9.

The electrodes 8 and 9 of each group 4a, 4b or 5a, 5b are associated to at least one respective cursor 16 or 17 made of dielectric material and moved by respective means 14 and 15 towards the cursor 17 or 16 facing it. Each cursor 16 and 17 exhibits a pair of horizontal slots 29, 30 freely crossed by respective pivots 31 and 32 constrained to vertical bars 33 and 34 which bars are inferiorly fixed to a slide pair 18 and 19. The slides 18 and 19 are supported by a support structure 20 and are mobile, in approaching or distancing, along a direction which is perpendicular to the slide bars 27.

Between each slide 18 and 19 and the cursors 4a and 4b and respectively 5a and 5b movement means for the said cursors are envisaged, which movement means are denoted by 14 and 15 and represented by two fluid-dynamic cylinders in figures 1 and 2. The slides 18 and 19 are also subject to the action of respective movement means 23 and 24, represented in the figures by a couple of fluid-dynamic cylinders, anchored on one side to the respective slide 18 or 19 and on the other side to a cross-bar 201 of the support structure 20, which cross-bar 201 is arranged in a substantially central position to the support structure 20. All the fluid-dynamic cylinders 14 and 15 and 23 and 24 are commanded by the processing unit 7 through respective power stages 35. The cylinders 14 and 15 differ from the cylinders 23 and 24 in that the force they exert on the cursors 16 and 17 is less than that exerted by the cylinders 23 and 24 on the slides 18 and 19 for a reason which will become more evident during the course of this description.

Above the cross-bar 201 a structure (not illustrated) is envisaged which supports evidencing means 6 constituted, in the embodiment herein described, by a plurality of spraying nozzles 25 fed by a reservoir (not illustrated) and commanded by the processing unit 7. The nozzles can be envisaged in an equal number to the number of electrodes 8 or 9 constituting each, group 4 or 5 or, preferably, in a greater number for the greater precision of the device 1 of the present invention. For example, a principal nozzle 25p could be envisaged for each electrode 8 or 9 and another, interpolating nozzle 25i between each principal nozzle 25p: the nozzles 25p are coplanar to the respective electrode pairs 8-9.

With reference in particular to figure 4, it may be noted that at the outlets of the process unit 7 the following are connected: the impulse generator 10, the selectors 12 and 13, the power stages 35, the impulse receiver 11 and the fluid-dynamic cylinders 14 and 15 and 23 and 24. To an inlet of the same processing unit 7 is connected the impulse receiver 11 through all analogue-to-digital converter 36. Other elements not indicated in the figure are connected to the processing unit 7, which elements are able to provide the said processing unit 7 with information inherent to the functioning conditions of the unit.

The functioning of the device is as follows.

A limb 2 is placed on the plane 26 and the processing unit 7 is operated on to command the activation of the device 1 after having, if necessary, supplied any relevant corrective parameters. The processing unit 7, which at the termination of the preceding phase had distanced the slides 18 and 19 from each other and the cursors 16 and 17 from each other, as well as the plane 26 from the structure 20, now brings the plane 26 towards the structure 20. Then it bring the slides 18 and 19 towards each other up until the electrodes 8 and 9 come almost into contact with the limb 2. Then the fluid-dynamic cylinders 14 and 15 are activated, to bring the electrodes 8 and 9 into contact with the limb 2. In this way, the cursors 16 and 17 act as jaws with regard to the limb 2 and cannot squeeze with too much force and damage the limb 2. The processing unit 7 now selects one of the electrodes 8 and one of the electrodes 9 and activates the impulse generator 10. Contemporaneously the unit 7 reads the signal sent by the electrode 9 to the receiver 11 and memorises it in an element of a matrix generated internally to itself. The signal received by the receiver 11 and transformed by the analogue-digital converter 36 is a resistance value, or value of current absorbed, through the limb 2 and according to the direction defined by the selected electrodes 8 and 9. Successively, through the selectors 12 and 13, it selects one by one each couple constituted by one electrode 8 and one electrode 9 and memorises the signal sent by whatever electrode couple 8 and 9 through the impulse receiver 11 and the analogue-digital converter 36 in a corresponding element of the same matrix. After having taken readings from all the electrode couples 8 and 9, the processing unit 7 re-examines the memorised matrix and searches for the element of the said matrix which corresponds to the minimum value of absorbed resistance. On the basis of this value the processing unit 7 returns to the electrode couple 8 and 9 which provided that minimum value and activates the nozzle 25 situated along the direction defined by the resulting electrode couple 8 and 9, that is, at the intersection between the original direction and the median direction between the groups 4 and 5 of electrodes 8 and 9. If there are also nozzles 25i present, the processing unit 7 will execute an interpolation and activate one of the principal nozzles 25p or one of the interpolated nozzles 25i.

Since the minimum resistance value is at the joint, taking along the y axis the resistance 25 value measured and along the x axis the distance of each electrode measured along a parallel direction to that of the slide bars 27 and envisaging that the joint will be within the zone described by the electrodes 8 and 9, a diagram 05 traced will be substantially parabolic in shape, that is, it will have first a decreasing shape and then all increasing one, and two lateral maximums and one central minimum.

In order to obtain a greater efficiency of the device 1 according to the present invention, and a more secure and rapid identification of the joint, it may be envisaged that the processing unit 7 is both capable of generating and recognising the aforementioned curve and capable of activating the movement means 28 of the support plane 26 whenever the unit 7 reads that the said curve has a development which is only increasing or only decreasing, indicative of the fact that the joint is certainly not between the electrodes. In such cases, the processing unit 7 would distance the electrodes 4 and 5 and would bring the support plane 6 in the direction in which the said curve exhibits its minimum value; then the unit 7 would bring the sensors 4 and 5 towards each other again and would take another reading with a possible successive movement of the support plane 26 until it reads a substantially parabolic curve.

The advantages of the invention are evident in that it permits of identifying the joint surely, rapidly and objectively.

A further advantage of the invention consists in the possibility of taking into account, automatically by means of sensors connected to the processing unit 7 or manually by means of the insertion of data into the processing unit 7, of parameters which might influence the operation, such as the environmental conditions of the limb 2, which might be deep-frozen. In cases where the limb 2 is indeed deep-frozen, manual identification of the joint is almost impossible since the joint is so rigid that it could not be manually compressed.

Numerous modifications and variations could be made to the invention, without foresaking the inventive idea it contains. For example, the sensors could be of various types and able to emit any kind of impulse and read any type of physical characteristic or dimension. Furthermore, evidencing means elements could be used which are capable of applying labels or similar on the limbs.

## Claims

1. A device for identifying and evidencing distinct anatomical regions in animals, in particular specific points in animal limb joints, comprising means of support (3) at least of the animal part or limb (2) in which a distinct anatomical region or specific joint point is to be found, at least two sensors (4,5) arranged and operating on said limb (2) being able to obtain at least a characteristics or physical size of the said anatomical region of the animal or limb (2) to be identified, evidencing means (6) for evidencing the said identified anatomical region or points of the said region, a processing unit (7) placed in communication with the said sensors (4, 5) and the said evidencing means (6) and able to process the data received from the said sensors (4, 5) and to command the activation of the said evidencing means (6) in relation to the data provided by the said sensors (4, 5), **characterised by the fact that** the said sensors (4, 5) each constituted by a plurality of respective electrodes (8, 9) placed side-by-side and constituting two groups of sensors (4, 5) destined to come into contact, on opposite sides, with the said limb (2), each group of sensors (4, 5) is borne by at least one respective cursor (16, 17) made of dielectric material moved by respective means (14, 15) to distance the said sensors (4, 5) from each other and also to bring them towards each other, said electrodes (8, 9) connected, in the case of the one sensor group (4), to an impulse generator (10) and, in the case of the other sensor group (5), to an impulse receiver (11); the said impulse generator (10), being connected to an outlet of the said processing unit (7) and the said impulse receiver (11) being connected to an inlet of the said processing unit (7); there being envisaged between the electrodes (8, 9) of each group of sensors (4, 5) and the said impulse generator (10) and respectively the said impulse receiver (11) a respective selector (12, 13) commanded by the said processing unit (7) and aimed at connecting only one of the said electrodes (8, 9) to the said impulse generator (10) and respectively to the said impulse receiver (11); the said processing units (7) being able to command the said selectors (12, 13) and, at the same time, able to activate, at every communication between an electrode (8) of one sensor (4) and an electrode (9) of the other sensor group (5), both the said impulse generator (10) and the said impulse receiver (11) for the determination of the minimum transversal resistance or minimum current absorbed and to command the said evidencing means (6) to make a distinctive sign on the said limb (2) in a region substantially identified by the median axis between the said group of electrodes (4, 5) and by the direction defined by the electrodes (8, 9) between which the said minimum transversal resistance or minimum current absorbed was read.

2. A device as in claim 1, wherein the said electrodes (8, 9) each have a substantially halfmoon conformation, the electrodes (8, 9) of the said sensors (4, 5) being arranged with their respective concave tracts (21, 22) reciprocally facing and having a curvature which decreases gradually from the said support means (3) onwards, in such a way as to increase the contact surface between the said concave tracts (21, 22) of the electrodes (8, 9) and the said animal limb (2).

3. A device as in claim 1, wherein each said cursor (16, 17) is borne by a respective slide (18, 19) mobile along a support structure (20) both in reciprocal approaching and distancing by the action of movement means (23, 24); the movement means (14, 15) relative to the said cursors (16, 17) exerting on the said cursors (16, 17) an approaching force which is inferior to that exerted by the movement means (23, 24) relative to the said slides (18, 19).

4. A device as in claim 1, wherein the said evidencing means (6) are constituted by a plurality of nozzles (25) each able to feed a certain quantity of ink on to the animal limb (2) on the command of the said processing unit (7).

5. A device as in claim 1, wherein the said evidencing means (6) are constituted by a plurality of nozzles (25), each able to transfer a mark on the said animal limb (2) on the command of the said processing unit (7).

6. A device as in claim 1, wherein the said support means (3) of the said animal limb (2) are constituted by a support plane (26) slidably mounted on respective guide bars (27) and moved along them in either direction by respective movement means (28) commanded by the said processing unit (7).

## Patentansprüche

1. Vorrichtung zur Erkennung und Markierung von bestimmten anatomischer Bereichen bei Tieren, insbesondere von spezifischen Stellen an tierischen Gliedgelenken, enthaltend Mittel zum Halten (3) von wenigstens dein tierischen Teil oder des Gliedes (2), an welchem ein bestimmter anatomischer Bereich oder spezifischer Gelenkpunkt gefunden wurde; wenigstens zwei Abtaster (4, 5), die an dem genannten Glied (2) positioniert sind, an diesem arbeiten und in der Lage sind, wenigstens eine typische oder physische Grösse des genannten anatomischen Bereiches des zu erkennenden tierischen Gliedes (2) zu erfassen; Markierungsmittel (6) zum Markieren des genannten erkannten anatomischen Bereiches oder der Stellen des genannten Bereiches; eine Rechnereinheit (7), angeordnet in Verbindung mit den genannten Abtastern (4, 5) und den genannten Markierungsmitteln (6) und in der Lage, die von den genannten Abtastern (4, 5) empfangenen Daten zu verarbeiten und die Aktivierung der genannten Markierungsmittel (6) im Verhältnis zu den Daten auszulösen, die von den genannten Abtastern (4, 5) geliefert werden, **dadurch gekennzeichnet**, dass die genannten Abtaster (4, 5) jeweils aus einer Anzahl von entsprechenden Elektroden (8, 9) bestehen, die Seite an Seite angeordnet und zwei Abtastergruppen (4, 5) bilden und dazu bestimmt sind, auf sich gegenüberliegenden Seiten mit dem genannten Glied (2) in Kontakt zu kommen, dabei wird jede Abtastergruppe (4, 5) von wenigstens einem entsprechenden Schieber (16, 17) aus dielektrischem Material getragen, der durch entsprechende Mittel (14, 15) bewegt wird, um die genannten Abtaster (4, 5) voneinander zu entfernen oder sie aufeinander zuzuführen, wobei die genannten Elektroden (8, 9) im Falle der einen Abtastergruppe (4) an einen Impulserzeuger (10) angeschlossen sind und im Falle der anderen Abtastergruppe (5) an einen Impulsempfänger (11); wobei der genannte Impulserzeuger (10) an einen Ausgang der genannten Rechnereinheit (7) angeschlossen ist und der genannte Impulsempfänger (11) an einen Eingang der genannten Rechnereinheit (7); wobei zwischen den Elektroden (8, 9) einer jeden Abtastergruppe (4, 5) und dein genannten Impulserzeuger (10) beziehungsweise dem genannten Impulsempfänger (11) ein entsprechender Wähler (12, 13) angeordnet ist, betätigt durch die genannte Rechnereinheit (7) und dazu bestimmt, nur eine der genannten Elektroden (8, 9) mit dem genannten Impulserzeuger (10) beziehungsweise dein genannten Impulsempfänger (11) zu verbinden; wobei die genannten Rechnereinheiten (7) in der Lage sind, die genannten Wähler (12, 13) zu steuern und gleichzeitig bei jeder Verbindung zwischen einer Elektrode (8) von einer Abtastergruppe (4) und einer Elektrode (9) der anderen Abtastergruppe (5) sei es den genannten Impulserzeuger (10) wie auch den genannten Impulsempfänger (11) zu aktivieren, und zwar zur Bestimmung des geringsten Querwiderstandes oder der geringsten Stromaufnahme sowie zum Auslösen der genannten Markierungsmittel (6), um ein deutliches Markierungszeichen an dem genannten Glied (2) in einem Bereich anzubringen, der im wesentlichen durch die mittlere Achse zwischen den genannten Abtastergruppen (4, 5) und durch die Richtung erkannt ist, die von der genannten Elektrodengruppe (8, 9) beschrieben ist, zwischen denen der genannte geringste Querwiderstand oder die geringste Stromaufnahme gelesen wurde.

2. Vorrichtung nach Patentanspruch 1, **dadurch gekennzeichnet**, dass die genannten Elektroden (8, 9) jeweils eine im wesentlichen halbmondförmige Ausbildung haben, wobei die Eleketroden (8, 9) der genannten Abtaster (4, 5) mit ihren entsprechenden konkaven Abschnitten (21, 22) einander zugewandt angeordnet sind und einen Bogen aufweisen, der allmählich von den genannten Haltemitteln (3) aus nach oben hin abnimmt, und zwar auf solche Weise, dass die Kontaktfläche zwischen den genannten konkaven Abschnitten (21, 22) der Elektroden (8, 9) und dem genannten tierischen Glied (2) zunimmt.

3. Vorrichtung nach Patentanspruch 1, **dadurch gekennzeichnet**, dass jeder der genannten Schieber (16, 17) von einem entsprechenden Schlitten (18, 19) getragen wird, die beide entlang einer Trägerstruktur (20) in gegenseitiger Annäherung oder in Entfernung voneinander beweglich sind, und zwar durch die Wirkung von Antriebsmitteln (23, 24); wobei die sich auf die genannten Schieber (16, 17) beziehenden Antriebsmittel (14, 15) auf die genannten Schieber (16, 17) eine Schubkraft ausüben, die geringer ist als die, welche die Antriebsmittel (23, 24) ausüben, die sich auf die genannten Schlitten (18, 19) beziehen.

4. Vorrichtung nach Patentanspruch 1, **dadurch gekennzeichnet**, dass die genannten Markierungsmittel (6) aus einer Anzahl von Düsen (25) bestehen, von denen jede in der Lage ist, auf den Befehl der genannten Rechnereinheit (7) hin eine bestimmte Menge von Farbe auf das tierische Glied (2) abzugeben.

5. Vorrichtung nach Patentanspruch 1, **dadurch gekennzeichnet**, dass die genannten Markierungsmittel (6) aus einer Anzahl von Düsen (25) bestehen, von denen jede in der Lage ist, auf den Befehl der genannten Rechnereinheit (7) hin eine Markierung auf das genannte tierische Glied (2) zu übertragen.

6. Vorrichtung nach Patentanspruch 1, **dadurch gekennzeichnet**, dass die genannten Haltemittel (3) für das genannte tierische Glied (2) aus einer Auflagefläche (26), die gleitbar auf entsprechenden Führungsschienen (27) montiert ist und entlang diesen in jeder Richtung durch entsprechende und von der genannten Rechnereinheit (7) ausgelösten Antriebsmittel (28) bewegt wird.

## Revendications

1. Appareil pour identifier et marquer des zones anatomiques distinctes d'animaux, en particulier des points spécifiques dans les joints des membres, comprenant un dispositif de support (3) au moins de la partie du membre (2) de l'animal dans laquelle une zone anatomique distincte ou un point spécifique du joint doit être localisé, au moins deux capteurs (4,5) mis en place et fonctionnant sur ledit membre (2), en mesure de permettre l'identification d'une caractéristique ou de la mesure physique de ladite zone anatomique de l'animal ou du membre (2), un dispositif de marquage (6) servant à marquer ladite zone anatomique ou des points de ladite zone identifiés, une unité de traitement (7) reliée aux dits capteurs (4,7) et audit dispositif de marquage (6), à même de traiter les informations reçues desdits capteurs (4,5) et de commander l'activation dudit dispositif de marquage (6) en fonction des informations fournies par lesdits capteurs (4,5), caractérisé en ce que lesdits capteurs (4,5), dont chacun est constitué d'un ensemble d'électrodes (8,9) placées côte-à-côte et constituant deux groupes de capteurs (4,5) destinés à entrer en contact, de côtés opposés, avec ledit membre (2), chaque groupe de capteurs (4,5) est supporté par au moins un curseur (16, 17), fabriqué dans un matériau diélectrique, mû par un dispositif (14, 15) pour éloigner lesdits capteurs (4,5) l'un de l'autre et pour les rapprocher, lesdites électrodes (8,9) reliés, dans le cas d'un groupe capteur (4), à un générateur d'impulsions (10) et, dans le cas de l'autre groupe capteur (5), à un récepteur d'impulsions (11) ; ledit générateur d'impulsions (10), étant relié à une sortie de ladite unité de traitement (7) et ledit récepteur d'impulsions (11) étant relié à une entrée de ladite unité de traitement (7); étant prévu entre les électrodes (8,9) de chaque groupe de capteurs (4,5) et le dit générateur d'impulsions (10) d'une part et ledit récepteur d'impulsions (11) un sélecteur (12, 13) commandé par ladite unité de traitement (7) et destiné à relier uniquement l'une desdites électrodes (8,9) audit générateur d'impulsions (10) et audit récepteur d'impulsions (11); ladite unité de traitement (7) étant en mesure de commander lesdits sélecteurs (12, 13) et, en même temps, d'activer, à chaque communication entre l'électrode (8) de l'un des capteurs (4) et l'électrode (9) de l'autre groupe capteur (5), ledit générateur d'impulsions (10) et ledit récepteur d'impulsions (11) pour la détermination de la résistance transversale minimale ou du courant absorbé minimal et de commander ledit dispositif de marquage (6) pour faire un signe distinctif sur ledit membre (2), dans une zone substantiellement identifiée par l'axe médian entre ledit groupe d'électrodes (4,5) et par la direction définie par les électrodes (8,9) entre lesquels ladite résistance transversale minimale ou ledit courant absorbé minimal a été lu.

2. Un appareil tel qu'à la revendication 1, dans lequel lesdites électrodes (8,9) présentent approximativement une conformation en demie-lune, les électrodes (8,9) desdits capteurs (4,5) étant disposés de façon telle que leurs faces concaves (21, 22) se font face réciproquement et présentent une courbe qui décroît graduellement en s éloignant dudit support (3) supérieur, de façon à augmenter la surface de contact entre lesdites faces concaves (21,22) des électrodes (8,9) et ledit membre d'animal (2).

3. Un appareil tel qu'à la revendication 1, dans lequel chacun desdits curseurs (16,17) est supporté par un coulisseau (18, 19) se déplaçant sur une structure de support (20), par rapprochement et éloignement réciproque, sous l'action du dispositif de mouvement (23, 24) ; le dispositif de mouvement (14, 15) relatif auxdits curseurs (16,17) exerçant sur lesdits curseurs une force de rapprochement inférieure à la force exercée par les dispositif de mouvement (23,24) relatif auxdits coulisseaux (18, 19).

4. Un appareil tel qu'à la revendication 1, dans lequel ledit dispositif de marquage (6) est constitué d'un ensemble de diffuseurs (25) dont chacun est en mesure d'injecter une certaine quantité d'encre sur le membre (2) de l'animal, sur commande de ladite unité de traitement (7).

5. Un appareil tel qu'à la revendication 1, dans lequel ledit dispositif de marquage (6) est constitué d'un ensemble de diffuseurs (25), dont chacun est en mesure d'apposer une marque sur ledit membre (2) de l'animal, sur commande de ladite unité de traitement (7).

6. Un appareil tel qu'à la revendication 1, dans lequel ledit support (3) dudit membre (2) d'animal est constitué d'un plateau de support (26) coulissant sur des glissières (27), mû dans chaque direction par un dispositif de mouvement (28) commandé par ladite unité de traitement (7),
